# EUROPEAN PATENT SPECIFICATION

(11) **EP 0 365 119 B1**
(45) Date of publication and mention of the grant of the patent: **30.11.1994**
(21) Application number: 89308116.6
(22) Date of filing: 09.08.1989
(51) Int. Cl.: A61K 9/72, A61K 31/47

(54) **Pharmaceutical composition**
Arzneimittel
Composition pharmaceutique

(30) Priority: 27.08.1988 GB 8820398
(43) Date of publication of application: 25.04.1990
(73) Proprietor: FISONS plc, Ipswich Suffolk IP1 1QH (GB)
(72) Inventor: Brown, Kenneth, Loughborough Leicestershire (GB)
(74) Representative: Craig, Christopher Bradberry

(56) References cited:
- EP-A- 0 287 193
- GB-A- 2 157 291
- GB-A- 2 187 953
- US-A- 3 957 965
- LACHMAN, et al. "The Theory and Practice of Industrial Pharmacy", 3rd edition, 1986 LEA & FEBIGER, Philadelphia, page 326,483, 489, 603.
- "Hawley's Condensed Chemical Dictionary", eleventh edition, 1987, VAN NOSTRAND REINHOLD, New York, page 24
- "Stedman's medical dictionary", 23rd edition,, The Williams & Wilkins Company, Baltimore, page 26
- "MARTINDALE, The Extra Pharmacopoeia", 29th edition, 1989, THE PHARMACEUTICAL PRESS, London, page 1069
- Eur. J. Respir. Dis. vol. 69, suppl.147, 1986, pages 149-159, S.T. Holgate "Clinical evaluation of nedocromil sodium in asthma"
- Aust. New Zealand J. Med, vol, 17, no. 6, 1987, R.E. Ruffin et al "The efficacy of medocromil sodium (Tilade) in asthma"
- Thorax, vol. 39, 6th June 1984, pages 809-812, S.Lal et al, "Nedocromil sodium : a new drug for the management of bronchial asthma"

## Description

This invention relates to pharmaceutical formulations, in particular to aerosol formulations for inhalation.

Many drugs, especially those for the treatment of diseases of the respiratory tract, are administered by inhalation. For administration by this route, powdered inhalation drugs are commonly formulated as pressurised aerosols. Nedocromil sodium, the disodium salt of 9-ethyl-6,9-dihydro-4,6-dioxo-10-propyl-4H-pyrano[3,2-g]quinoline-2,8-dicarboxylic acid, is known to be effective in the treatment of inter alia reversible obstructive airways disease when administered by inhalation. Indeed, pressurised aerosol formulations of nedocromil sodium are known, for example from British Patent Application No 2 157 291 A, published 23 October 1985. However, it has been found that certain patients do not derive the full expected therapeutic benefit from the medicament.

We have now found that the incorporation into a pressurised aerosol formulation of nedocromil sodium of a flavouring agent reduces or substantially overcomes this problem.

Thus, according to the invention there is provided a pharmaceutical pressurised aerosol inhalation composition comprising, as active ingredient, 9-ethyl-6,9-dihydro-4,6-dioxo-10-propyl-4H-pyrano[3,2-g]-quinoline-2,8-dicarboxylic acid or a pharmaceutically acceptable salt thereof, having a mass median diameter in the range 0.01 to 10 µm, a pressurised or liquefied gas propellant, and a flavouring agent selected from the group consisting of peppermint oil and menthol.

The composition according to the invention is useful in the treatment of reversible obstructive airways disease and is advantageous in that it induces improved patient compliance resulting in more regular use of the medicament with concomitant improvements in the therapeutic benefits to the patient. These effects are especially significant in the case of middle-aged and younger patients whose disease has generally not reached an advanced stage in terms of duration, severity and reversibility.

The composition is also advantageous in that it may have an improved 'dispersion' ie may produce a higher proportion of medicament particles which are fine enough to penetrate deep into the lung. In addition, aerosol dispensers containing the compositions of the invention may be less prone to blockage than are containers of known formulations of the active ingredient. Valve action may also be improved by the presence of the flavouring agents, which may give rise, for example, to reduced leakage and/or greater dose uniformity.

Pharmaceutically acceptable salts of 9-ethyl-6,9-dihydro-4,6-dioxo-10-propyl-4H-pyrano[3,2-g]quinoline -2,8-dicarboxylic acid include the alkali metal salts and the alkaline earth salts. We particularly prefer the disodium salt, which is known by the generic name nedocromil sodium.

A variety of flavouring agents may be used in the composition according to the invention. The proprietary product known by the tradename Dentomint, which contains both menthol and peppermint oil, may also be used.

The composition may also contain a sweetening agent. Suitable sweetening agents include sugar, aspartame, cyclamates and, preferably, saccharin or salts thereof, eg saccharin sodium.

We prefer the composition to include both a flavouring agent and a sweetening agent.

The active ingredient has a mass median diameter in the range 0.01 to 10 microns, more preferably from 1 to 5 microns. The composition preferably comprises from 0.05 to 15, preferably from 0.1 to 10, and most preferably from 0.5 to 5% w/w active ingredient.

We prefer the flavouring and sweetening agents, when they are solid, to have a mass median diameter in the range 0.01 to 10 µm. The composition preferably comprises up to 5% w/w, more preferably up to 2% w/w, flavouring and sweetening agents.

The pressurised or liquefied gas propellant may be any of the propellants conventionally used in pressurised aerosols. For example, the propellant may be a chlorofluorocarbon such as those known as trichlorofluoromethane (Propellant 11), dichlorodifluoromethane (Propellant 12) and 1,2-dichloro-1,1,2,2-tetrafluoroethane (Propellant 114) or a mixture of two or more thereof. In this case, we prefer the propellant to be a mixture of Propellant 12 and Propellant 114. In particular, we prefer the ratio of Propellant 12 to Propellant 114 to be from about 1:1 to about 2:1, especially about 1.5:1 ie we prefer an excess of Propellant 12 over Propellant 114.

Alternatively, the propellant may be a hydrofluorocarbon, eg that known as HFC 134a. Other propellants which may be used are compressed gases, eg nitrogen and hydrocarbons such as butane.

The composition may also include a surface active agent. The surface active agent may be solid or, more preferably, liquid. The surface active agent is preferably non-ionic. Non-ionic liquid surface active agents which may be mentioned include the oleates of sorbitan. We particularly prefer the surface active agent to be sorbitan trioleate.

The compositions according to the invention preferably contain less than 1.0%, more preferably less than 0.5% and most preferably less than 0.2% w/w of water.

The compositions according to the invention may be prepared by conventional techniques. Generally, all that is required is mixing of the ingredients. Where the propellant is a mixture of Propellant 12 and Propellant 114, the compositions are preferably prepared by mixing the solid components in a pressure vessel, evacuating the vessel and then admitting the propellant mixture.

The compositions according to the invention may be packaged in conventional pressurised aerosol canisters, eg aluminium or glass canisters fitted with valves, preferably metering valves, to permit accurate dispensing of aliquots of the composition.

The invention is illustrated, but in no way limited, by the following Examples.

### Example 1

| Ingredients | % w/w |
|---|---|
| Nedocromil sodium (micronised) (calculated as anhydrous material) | 1.442 |
| Sorbitan trioleate | 0.5 |
| Menthol BP | 0.05 |
| Saccharin sodium BP (micronised) | 0.033 |
| Propellant mixture 12/114 (60/40% w/w) | 97.975 |

### Method

The sorbitan trioleate, menthol, saccharin sodium (dried and micronised) and nedocromil sodium (micronised) are weighed into a clean, dry batching vessel. The batching vessel is sealed, evacuated and filtered, and the propellant mixture admitted to the required weight.

The mixture is stirred and filled into aluminium aerosol cans fitted with 100 µl metering valves.

### Example 2

| Ingredients | % w/w |
|---|---|
| Nedocromil sodium (micronised) (calculated as anhydrous material) | 1.442 |
| Sorbitan trioleate | 0.5 |
| Dentomint | 0.577 |
| Saccharin sodium BP (micronised) | 0.072 |
| Propellant mixture 12/114 (60/40% w/w) | 97.409 |

### Method

Prepared by the method of Example 1.

### Example 3

| Ingredients | % w/w |
|---|---|
| Nedocromil sodium (micronised) (calculated as anhydrous material) | 1.442 |
| Sorbitan trioleate | 0.5 |
| Dentomint | 0.288 |
| Saccharin sodium BP (micronised) | 0.036 |
| Propellant mixture 12/114 (60/40% w/w) | 97.734 |

### Method

Prepared by the method of Example 1.

## Claims (Claims for the following Contracting State(s): AT, BE, CH, DE, FR, GB, IT, LI, LU, NL, SE)

1. A pharmaceutical pressurised aerosol inhalation composition comprising, as active ingredient, 9-ethyl-6,9-dihydro-4,6-dioxo-10-propyl-4H-pyrano[3,2-g]-quinoline-2,8-dicarboxylic acid or a pharmaceutically acceptable salt thereof, having a mass median diameter in the range 0.01 to 10 µm, a pressurised or liquefied gas propellant, and a flavouring agent selected from the group consisting of peppermint oil and menthol.

2. A composition according to Claim 1, wherein the active ingredient is nedocromil sodium.

3. A composition according to Claim 1 or 2, which includes a sweetening agent.

4. A composition according to Claim 3, wherein the sweetening agent is selected from the group consisting of sugar, aspartame, cyclamates and saccharin or salts thereof.

5. A composition according to any one of the preceding claims, wherein the propellant is a mixture of dichlorodifluoromethane (Propellant 12) and 1,2-dichloro-1,1,2,2-tetrafluoroethane (Propellant 114).

6. A composition according to Claim 5, wherein the ratio of dichlorodifluoromethane to 1,2-dichloro-1,1,2,2-tetrafluoroethane is from 1:1 to 2:1.

7. A pressurised aerosol canister containing a composition according to any one of the preceding claims and fitted with a dispensing metering valve.

## Claims (Claims for the following Contracting State(s): ES and GR)

1. A process for the preparation of a pharmaceutical pressurised aerosol inhalation composition comprising, as active ingredient, 9-ethyl-6,9-dihydro-4,6-dioxo-10-propyl-4H-pyrano[3,2-g]quinoline-2,8-dicarboxylic acid or a pharmaceutically acceptable salt thereof, having a mass median diameter in the range 0.01 to 10 µm, a pressurised or liquefied gas propellant, and a flavouring agent selected from the group consisting of peppermint oil and menthol, which process comprises mixing the ingredients.

2. A process according to Claim 1, wherein the active ingredient is nedocromil sodium.

3. A process according to Claim 1 or 2, wherein the composition includes a sweetening agent.

4. A process according to any Claim 3, wherein the sweetening agent is selected from the group consisting of sugar, aspartame, cyclamates and saccharin or salts thereof.

5. A process according to any one of the preceding claims, wherein the propellant is a mixture of dichlorodifluoromethane (Propellant 12) and 1,2-dichloro-1,1,2,2-tetrafluoroethane (Propellant 114).

6. A process according to Claim 5, wherein the ratio of dichlorodifluoromethane to 1,2-dichloro-1,1,2,2-tetrafluoroethane is from 1:1 to 2:1.

7. A pressurised aerosol canister containing a composition prepared in accordance with any one of the preceding claims and fitted with a dispensing metering valve.

## Patentansprüche (Patentansprüche für folgende(n) Vertragsstaat(en): AT, BE, CH, DE, FR, GB, IT, LI, LU, NL, SE)

1. Pharmazeutische, unter Druck gesetzte Aerosol-Inhalationszusammensetzung, welche, als aktiven Bestandteil, 9-Ethyl-6,9-dihydro-4,6-dioxo-10-propyl-4H-pyrano[3,2-g]chinolin-2,8-dicarbonsäure oder ein pharmazeutisch annehmbares Salz hievon mit einem mittleren Massedurchmesser im Bereich von 0,01 bis 10 µm, ein unter Druck gesetztes oder verflüssigtes Gastreibmittel und einen Geschmacksstoff, ausgewählt aus der Gruppe bestehend aus Pfefferminzöl und Menthol, umfaßt.

2. Zusammensetzung nach Anspruch 1, worin der aktive Bestandteil Natriumnedocromil ist.

3. Zusammensetzung nach Anspruch 1 oder 2, welche einen Süßstoff enthält.

4. Zusammensetzung nach Anspruch 3, worin der Süßstoff aus der Gruppe bestehend aus Zucker, Aspartam, Cyclamaten und Saccharin oder Salzen hievon ausgewählt ist.

5. Zusammensetzung nach einem der vorhergehenden Ansprüche, worin das Treibmittel eine Mischung von Dichlordifluormethan (Treibmittel 12) und 1,2-Dichlor-1,1,2,2-tetrafluorethan (Treibmittel 114) ist.

6. Zusammensetzung nach Anspruch 5, worin das Verhältnis von Dichlordifluormethan zu 1,2-Dichlor-1,1,2,2-tetrafluorethan 1:1 bis 2:1 beträgt.

7. Unter Druck gesetzter Aerosol-Behälter, welcher eine Zusammensetzung nach einem der vorhergehenden Ansprüche enthält und mit einem Abgabemeßventil ausgestattet ist.

## Patentansprüche (Patentansprüche für folgende(n) Vertragsstaat(en): ES, GR)

1. Verfahren zur Herstellung einer pharmazeutischen, unter Druck gesetzten Aaerosol-Inhalationszusammensetzung, die, als aktiven Bestandteil, 9-Ethyl-6,9-dihydro-4,6-dioxo-10-propyl-4H-pyrano[3,2-g]chinolin-2,8-dicarbonsäure oder ein pharmazeutisch annehmbares Salz hievon mit einem mittleren Massedurchmesser im Bereich von 0,01 bis 10 µm, ein unter Druck gesetztes oder verflüssigtes Gastreibmittel und einen Geschmacksstoff, ausgewählt aus der Gruppe bestehend aus Pfefferminzöl und Menthol, umfaßt, welches Verfahren das Mischen der Bestandteile umfaßt.

2. Verfahren nach Anspruch 1, bei welchem der aktive Bestandteil Natriumnedocromil ist.

3. Verfahren nach Anspruch 1 oder 2, bei welchem die Zusammensetzung einen Süßstoff enthält.

4. Verfahren nach Anspruch 3, bei welchem der Süßstoff aus der Gruppe bestehend aus Zucker, Aspartam, Cyclamaten und Saccharin oder Salzen hievon ausgewählt wird.

5. Verfahren nach einem der vorhergehenden Ansprüche, bei welchem das Treibmittel eine Mischung von Dichlordifluormethan (Treibmittel 12) und 1,2-Dichlor-1,1,2,2-tetrafluorethan (Treibmittel 114) ist.

6. Verfahren nach Anspruch 5, bei welchem das Verhältnis von Dichlordifluormethan zu 1,2-Dichlor-1,1,2,2-tetrafluorethan 1:1 bis 2:1 beträgt.

7. Unter Druck gesetzter Aerosol-Behälter, welcher eine Zusammensetzung, hergestellt nach einem der vorhergehenden Ansprüche, enthält und mit einem Abgabemeßventil ausgestattet ist.

## Revendications (Revendications pour l'(les) Etat(s) contractant(s) suivant(s): AT, BE, CH, DE, FR, GB, IT, LI, LU, NL, SE)

1. Composition pharmaceutique d'un aérosol pressurisé pour inhalation comprenant, comme ingrédient actif, l'acide 9-éthyl-6,9-dihydro-4,6-dioxo-10-propyl-4H-pyrano[3,2-g]quinoléine-2,8-dicarboxylique ou un sel pharmaceutiquement acceptable de celui-ci, ayant un diamètre médian en masse dans l'intervalle de 0,01 à 10 µm, un agent propulseur pressurisé ou liquéfié et un agent aromatisant choisi parmi le groupe constitué de l'huile de menthe et du menthol.

2. Composition suivant la revendication 1, dans laquelle l'ingrédient actif est le nédocromil sodique.

3. Composition suivant la revendication 1 ou 2, qui comprend un agent édulcorant.

4. Composition suivant la revendication 3, dans laquelle l'agent édulcorant est choisi parmi le groupe constitué du sucre, de l'aspartame, des cyclamates et de la saccharine ou des sels de ceux-ci.

5. Composition suivant l'une quelconque des revendications précédentes, dans laquelle l'agent propulseur est un mélange de dichlorodifluorométhane ("Propellant 12") et de 1,2-dichloro-1,1,2,2-tétrafluoroéthane ("Propellant 114").

6. Composition suivant la revendication 5, dans laquelle le rapport du dichlorodifluorométhane au 1,2-dichloro-1,1,2,2-tétrafluoroéthane est de 1:1 à 2:1.

7. Récipient métallique préssurisé pour aérosol contenant une composition suivant l'une quelconque des revendications précédentes et équipé d'une valve doseuse d'administration.

## Revendications (Revendications pour l'(les) Etat(s) contractant(s) suivant(s): ES, GR)

1. Procédé de préparation d'une composition pharmaceutique d'un aérosol pressurisé pour inhalation comprenant, comme ingrédient actif, l'acide 9-éthyl-6,9-dihydro-4,6-dioxo-10-propyl-4H-pyrano[3,2-g]quinoléine-2,8-dicarboxylique ou un sel pharmaceutiquement acceptable de celui-ci, ayant un diamètre médian en masse dans l'intervalle de 0,01 à 10 µm, un agent propulseur pressurisé ou liquéfié et un agent aromatisant choisi parmi le groupe constitué de l'huile de menthe et du menthol, lequel procédé comprend le mélange des ingrédients.

2. Procédé suivant la revendication 1, dans lequel l'ingrédient actif est le nédocromil sodique.

3. Procédé suivant la revendication 1 ou 2, dans lequel la composition comprend un agent édulcorant.

4. Procédé suivant la revendication 3, dans lequel l'agent édulcorant est choisi parmi le groupe constitué du sucre, de l'aspartame, des cyclamates et de la saccharine ou des sels de ceux-ci.

5. Procédé suivant l'une quelconque des revendications précédentes, dans lequel l'agent propulseur est un mélange de dichlorodifluorométhane ("Propellant 12") et de 1,2-dichloro-1,1,2,2-tétrafluoroéthane ("Propellant 114").

6. Procédé suivant la revendication 5, dans lequel le rapport du dichlorodifluorométhane au 1,2-dichloro-1,1,2,2-tétrafluoroéthane est de 1:1 à 2:1.

7. Récipient métallique pressurisé pour aérosol contenant une composition préparée suivant l'une quelconque des revendications précédentes et équipé d'une valve doseuse d'administration.
